(19) Europäisches Patentamt
European Patent Office
Office européen des brevets

(11) Veröffentlichungsnummer: **0 232 889 A2**

# (12) EUROPÄISCHE PATENTANMELDUNG

(21) Anmeldenummer: **87101783.6**

(22) Anmeldetag: **09.02.87**

(51) Int. Cl.[4]: **C07D 249/08**, A01N 43/653

(30) Priorität: **14.02.86 DE 3604569**

(43) Veröffentlichungstag der Anmeldung: **19.08.87 Patentblatt 87/34**

(84) Benannte Vertragsstaaten: **AT BE CH DE ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Buschmann, Ernst, Dr.**
**Georg-Ludwig-Krebs-Strasse 10**
**D-6700 Ludwigshafen(DE)**
Erfinder: **Zeeh, Bernd, Dr.**
**Queichstrasse 5**
**D-6703 Limburgerhof(DE)**
Erfinder: **Meyer, Norbert, Dr.**
**Dossenheimer Weg 22**
**D-6802 Ladenburg(DE)**
Erfinder: **Jung, Johann, Prof. Dr.**
**Hardenburgstrasse 19**
**D-6703 Limburgerhof(DE)**
Erfinder: **Rademacher, Wilhelm, Dr.**
**Austrasse 1**
**D-6703 Limburgerhof(DE)**
Erfinder: **Pommer, Ernst-Heinrich, Dr.**
**Berliner Platz 6**
**D-6703 Limburgerhof(DE)**
Erfinder: **Ammermann, Eberhard, Dr.**
**Sachsenstrasse 3**
**D-6700 Ludwigshafen(DE)**

## (54) Threo-konfigurierte Triazolylcarbinole - Fungizide und Wachstumsregulatoren.

(57) Weitgehend reine Triazolylcarbinole der Formel I

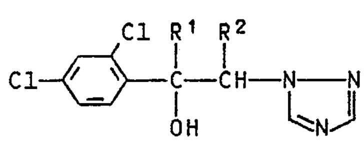

I,

in der
$R^1$ für einen Alkylrest und
$R^2$ für einen Alkylrest, für einen Cycloalkylrest, für einen Cycloalkylalkylrest, für Benzyl oder Phenyl steht, wobei die asymmetrischen Zentren des Moleküls RR/SS bzw. threo-konfiguriert sind und diese enthaltende Fungzide und wachstumsregulierende Mittel.

EP 0 232 889 A2

## Threo-konfigurierte Triazolylcarbinole -Fungizide und Wachstumsregulatoren

Die vorliegende Erfindung betrifft Triazolylcarbinole von neuartiger Struktur, Verfahren zu ihrer Herstellung und die Verwendung dieser Substanzen zur Bekämpfung pathogener Pilze und zur Regulierung des Pflanzenwachstums.

Es ist bekannt, daß Triazolylcarbinole fungizide Eigenschaften besitzen (DE-26 54 890). Die Wirksamkeit der Produkte, insbesondere bei niedrigen Aufwandmengen ist unbefriedigend.

Es wurden nun Triazolylcarbinole der Formel I gefunden, in der

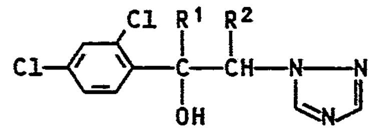

$R^1$ für einen $C_1$-$C_3$-Alkylrest,
$R^2$ für einen $C_1$-$C_8$-Alkylrest, für einen $C_3$-$C_8$-Cycloalkyl-$C_1$-$C_3$-alkylrest, für einen $C_3$-$C_8$-Cycloalkylrest, für gegebenenfalls substituiertes Benzyl oder Phenyl steht, wobei die asymmetrischen Zentren des Moleküls RR/SS bzw. threo-konfiguriert sind, die biologisch weitaus aktiver sind, als die in DE-OS 26 54 890 beschriebenen Triazolylcarbinole.

Weitgehend reine Verbindungen sind Verbindungen, die 90% (Gew.%) und mehr an dem RR/SS-Isomeren enthalten.

Die bekannte Umsetzung der Triazolylketone der Formel IV

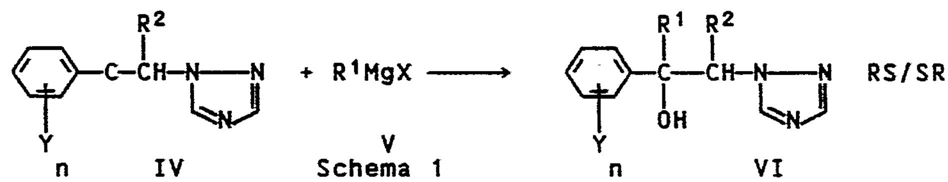

IV V Schema 1 VI

in der $R^2$ die oben genannten Bedeutungen haben kann und $Y_n$ für 2,4-$Cl_2$ stehen kann, mit Grignardreagenzien der Formel V in der $R^1$ und X die oben genannten Bedeutungen haben, führt wie in DE-26 54 890 beschrieben, zu einheitlichen Diastereomeren der Formel VI in der $R^1$, $R^2$ und $Y_n$ die oben genannten Bedeutungen haben und die asymmetrischen Zentren RS/SR bzw. erythro-konfiguriert sind. Diese entsprechend DE 26 54 890 erhaltenen Triazolylcarbinole sind als Fungizide und Wachstumsregulatoren weitaus weniger wirksam als die neuen Triazolylcarbinole der Formel I, die RR/SS bzw. threo-konfiguriert sind und die in hoher Diastereoselektivität durch Umsetzung der Triazolylketone III mit Grignardreagenzien II zugänglich sind.

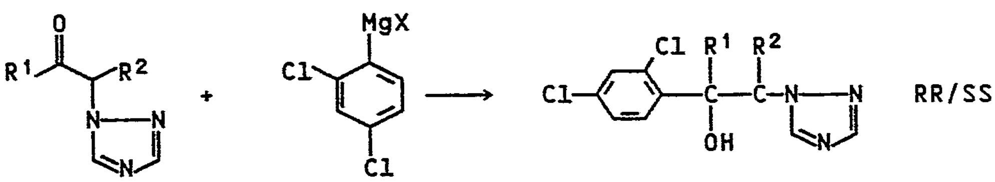

III II I

Schema 2

In Schema 2 haben $R^1$, $R^2$ und X die oben genannten Bedeutungen.

$R^1$ bedeutet z.B. Methyl, Ethyl, n-Propyl oder iso-Propyl,

$R^2$ bedeutet beispielsweise Methyl, Ethyl, n-Propyl, Isopropyl, n-Butyl, iso-Butyl, tert.-Butyl, n-Pentyl, iso-Pentyl, neo-Pentyl, n-Hexyl, Cyclopropyl, Cyclopentyl, Cyclohexyl, Cycloheptyl, Methylcyclohexyl, Methylcycloheptyl, Ethylcyclohexyl, Propylcyclohexyl, Benzyl, Halogenbenzyl, 4-Chlorbenzyl, 4-Fluorbenzyl, $C_1$-$C_4$-Alkylbenzyl, 4-$CH_3$-Benzyl, 4-$CF_3$-Benzyl, Phenyl, Halogenphenyl, 4-Chlorphenyl, 2,4-Dichlorphenyl, $C_1$-$C_4$-Alkoxyphenyl, 4-Methoxyphenyl.

Die Ausgangsverbindungen der Formel III sind aus DE 24 31 407 bekannt und können wie in DE 24 31 407 beschrieben hergestellt werden.

Die Umsetzung der Triazolylketone III mit den Grignardreagenzien der Formel II erfolgt, indem man die Ketone III in einem organischen Lösungsmittel, beispielsweise einem Ether, z.B. Di-n-butylether, Methyl-tert.-butylether, Diethylether, Tetrahydrofuran, oder einem aromatischen Kohlenwasserstoff, z.B. Toluol, Xylol, Benzol löst, diese Lösung vorlegt und die Suspension einer Grignardverbindung der Formel II in Tetrahydrofuran THF oder einem anderen Ether bei einer Temperatur von -10 bis 40°C zutropft. Dabei wird im wesentlichen das Triazolylcarbinol I gebildet.

Das folgende Beispiel erläutert die Darstellung der erfindungsgemäßen Triazolylcarbinole:

Zu 10,3 g 3-(1,2,4-Triazol-1-yl)-butan-2-on in 100 ml Ether wird eine Suspension von 0,1 Mol 2,4-Dichlorphenylmagnesiumjodid in 200 ml Ether zugetropft. Man erwärmt 2 h zum Rückfluß und hydrolysiert mit gesättigter wäßriger $NH_4Cl$-Lösung. Die organische Phase wird mit Wasser gewaschen, über $Na_2SO_4$ getrocknet und eingeengt. Das NMR-Spektrum des Rohproduktes zeigt die gewünschte Verbindung 1 (s. Tabelle) neben unumgesetzter Ausgangsverbindung. Die endgültige Reinigung erfolgt durch Chromatographie ($SiO_2$/$CH_2Cl_2$).

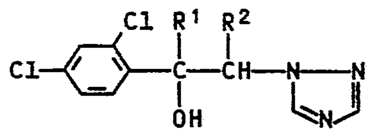

RR/SS

| Verbindung Nr. | $R^1$ | $R^2$ | Fp. °C | $^1$H-NMR δ-Werte $CDCl_3$ |
|---|---|---|---|---|
| 1 | $CH_3$ | $CH_3$ | | 1.71 (d, J = 7 Hz, 3H), 1.73 (s, 3H), 4.90 (s, 1H), 5.68 (q, J = 7 Hz, 1H), 7.05 (dd J = 7 + 3 Hz, 1H), 7.26 d, J = 3 Hz, 1H), 7.46 d, J = 7 Hz, 1H), 7,75 (s, 1H), 7.83 (s, 1H) |
| 2 | $CH_3$ | $C_2H_5$ | | 0.85 (t, J = 7 Hz, 3H 1,76 (s, 3H), 2.12 (m, 1H), 2.32 (m, 1H), 4.98 (s, 1H), 5.31 (d, d, J = 6 + 3 Hz, 1H), 7.03 (d, d, J = 7 + 3 Hz, 1H), 7.26 (d, J = 3 Hz, 1H), 7.47 (d, J = 7 Hz, 1H), 7.75 (s, 1H), 7.82 (s, 1H) |
| 3 | $CH_3$ | n-$C_3H_7$ | | |
| 4 | $CH_3$ | 2,2-Dimethyl-propyl-1 | | |
| 5 | $CH_3$ | Phenyl | | |
| 6 | $CH_3$ | Benzyl | | |
| 7 | $CH_3$ | Cyclohexyl | | |

| Vergleichssubstanzen | | Verbindung Nr. |
|---|---|---|
| 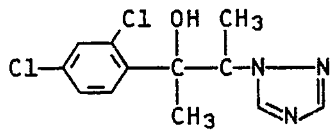 | RS/SR | $B_1$ |
| 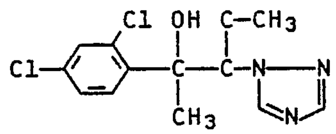 | RS/SR | $B_2$ |

$B_1$ und $B_2$ wurden entsprechend Schema 1, d.h. wie in DE 26 54 890 beschrieben, hergestellt.

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt-und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse -wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle,
Ustilago-Arten an Getreide und Zuckerrohr,
Venturia inaequalis (Schorf) an Äpfeln,
Septoria nodorum an Weizen,
Helminthosporium teres an Gerste,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Hemileia vastatrix an Kaffee,
Alternaria solani an Kartoffeln, Tomaten,
Plasmopara viticola an Reben sowie
Fusarium-und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden. Bei der Anwendung der Wirkstoffe im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Gra nulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 2 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III.20 Gew.-Teile der Verbindung Nr. 1 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 2 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 2 werden mit 97 Gew.-Teilen feinteiligem Kaolin innig vermischt. Man erhält auf diese Weise ein Stäubemittel, das 3 Gew.% des Wirkstoffs enthält.

VII.30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 2 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,

Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester

heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,

N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,

1-(4-Chlorphenoxy)-3,3-dimethyl-1-(1H-1,2,4-triazol-1-yl)-2-butanol,
1-(4-Phenylphenoxy)-3,3-dimethyl-1-(1H)-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4'-Difluor-α-(1H-1,2,4-triazolyl-1-methyl)-benzhydrylalkohol
N-(3-Chlor, 2,6-dinitro, 4-trifluormethylphenyl-5-trifluormethyl,
3-chlor, 2-aminopyridin,
1-((bis(4-Fluorphenyl)methylsilyl)-methyl-1H-1,2,4-triazol.

Anwendungsbeispiel 1

Wirksamkeit gegen Gerstenmehltau

Blätter von in Töpfen gewachsenen Gerstenkeimlingen der Sorte "Asse" mittel in der Trockensubstanz enthält, besprüht und nach 24 Stunden mit Oidien (Sporen) des Gerstenmehltaus (Erysiphe graminis f. sp. hordei) bestäubt. Die Versuchspflanzen werden anschließend im Gewächshaus bei Temperaturen zwischen 20 und 22°C und 75 bis 80% relativer Luftfeuchtigkeit aufgestellt. Nach 7 Tagen wird das Ausmaß der Mehltauentwicklung ermittelt.

Bewertung: 0 = kein Pilzbefall, abgestuft bis
5 = Totalbefall

| Wirkstoff Nr. | Befall der Blätter nach Applikation ... %iger Wirkstoffaufbereitung 0,006 | 0,0015 |
|---|---|---|
| 1 | 0 | 1 |
| $B_1$ | 3 | 3-4 |
| 2 | 0 | 1 |
| $B_2$ | 2 | 3-4 |
| Unbehandelt | 5 | |

Anwendungsbeispiel 2

Wirksamkeit gegen Gurkenmehltau (kurativ)

Junge Gurkenpflanzen der Sorte "Chinesische Schlange" werden im Zweiblattstadium mit einer wäßrigen Konidiensuspension des Gurkenmehltaus (Erysiphe cichoracearum und Sphaerotheca fuliginea) besprüht. Nach 3 Tagen werden diese Pflanzen mit wäßriger Spritzbrühe, die 80% Wirkstoff und 20% Emulgiermittel in der Trockensubstanz enthält, bis zur Tropfnässe besprüht und im Gewächshaus bei Temperaturen zwischen 20 und 22°C und 70 bis 80% Luftfeuchtigkeit aufgestellt. 21 Tage nach der Wirkstoffapplikation wird das Ausmaß des Pilzbefalls ermittelt.

Bewertung:

0 = kein Pilzbefall, abgestuft bis
5 = Totalbefall

A = leichter Blattschaden
B = mittlerer Blattschaden
C = starker Blattschaden
T = Totalschaden

| Wirkstoff Nr. | Befall der Blätter nach Applikation von 0,0125 %iger Wirkstoffaufbereitung |
|---|---|
| 1 | 0A |
| $B_1$ | 4 |
| 2 | 0 |
| $B_2$ | 3 |
| Unbehandelt | 5 |

Anwendungsbeispiel 3

Wirksamkeit gegen Weizenbraunrost

Blätter von in Töpfen gewachsenen Weizensämlingen der Sorte "Frühgold" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschließend mit wäßrigen Spritzbrühen, die 80% Wirkstoff und 20% Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70% relativer Luftfeuchte aufgestellt. Nach 8 Tagen wird das Ausmaß der Rostpilzentwicklung auf den Blättern ermittelt.

Bewertung:

0 = kein Pilzbefall, abgestuft bis
5 = Totalbefall

A = leichter Blattschaden
B = mittlerer Blattschaden
C = starker Blattschaden
T = Totalschaden

| Wirkstoff Nr. | Befall der Blätter nach Applikation von ... %iger Wirkstoffaufbereitung | | |
|---|---|---|---|
| | 0,025 | 0,006 | 0,0015 |
| 1 | 0A | 0A | 3 |
| $B_1$ | 3-4 | 4 | 4 |
| 2 | 0A | 2 | 3-4 |
| $B_2$ | 3-4 | 4 | 4 |
| Unbehandelt | | 5 | |

Anwendungsbeispiel 4

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80% Wirkstoff und 20% Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen ha sich die Krankheit auf den unbehandelten Kontrollpflanzen so stark entwickelt, daß die entstandenen Blattnekrosen den überwiegenden Teil der Blätter bedecken.

Bewertung: 0 = kein Pilzbefall, abgestuft bis
5 = Totalbefall

| Wirkstoff Nr. | Befall der Blätter nach Applikation von 0,05 %iger Wirkstoffaufbereitung |
|---|---|
| 1 | 2 |
| $B_1$ | 3-4 |
| 2 | 1 |
| $B_2$ | 3-4 |
| Unbehandelt | 5 |

Anwendungsbeispiel 5

Zur Bestimmung der wachstumsregulierenden Eigenschaften der Prüfsubstanzen wurden Testpflanzen auf ausreichend mit Nährstoffen versorgtem Kultursubstrat in Kunststoffgefäßen von ca. 12,5 cm Durchmesser angezogen.

Im Vorauflaufverfahren wurden die Testsubstanzen in wäßriger Aufbereitung am Tage der Einsaat auf das Saatbett gegossen.

Im Nachauflaufverfahren wurden die zu prüfenden Substänzen in wäßriger Aufbereitung auf die Pflanzen gesprüht. Die beobachtete wachstumsregulierende Wirkung wurde bei Versuchsende durch Wuchshöhenmessung belegt.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen zu entnehmen:

Vorauflauf-Bodenbehandlung: Gerste (Sorte Aramir)

| Wirkstoff | Wirkstoff mg/Gefäß | Wuchshöhen rel. |
|---|---|---|
| $B_1$ | 6 | 96,6 |
| $B_2$ | 6 | 90,8 |
| 1 | 6 | 30,9 |
| 2 | 6 | 69,6 |

Nachauflauf-Blattbehandlung: Gerste (Sorte Aramir)

| Wirkstoff | Wirkstoff mg/Gefäß | Wuchshöhen rel. |
|---|---|---|
| $B_1$ | 6 | 100 |
| 1 | 6 | 87,9 |

Nachauflauf-Blattbehandlung: Raps (Sorte Petronova)

| Wirkstoff | Wirkstoff mg/Gefäß | Wuchshöhen rel. |
|---|---|---|
| $B_1$ | 6 | 90,3* |
| 1 | 6 | 68,8 |

*Sproß deformiert und nekrotisch

Nachauflauf-Blattbehandlung: Weizen (Sorte Kolibri)

| Wirkstoff | Wirkstoff mg/Gefäß | Wuchshöhen rel. |
|---|---|---|
| $B_1$ | 6 | 96,5 |
| $B_2$ | 6 | 100,0 |
| 1 | 6 | 93,4 |
| 2 | 6 | 93,4 |

Vorauflauf-Bodenbehandlung: Raps (Sorte Petronova)

| Wirkstoff | Wirkstoff mg/Gefäß | Wuchshöhen rel. |
|---|---|---|
| $B_1$ | 6 | 86,4 |
| $B_2$ | 6 | 95,5 |
| 1 | 6 | 31,8 |
| 2 | 6 | 68,2 |

## Ansprüche

1. Weitgehend reine Triazolylcarbinole der Formel I

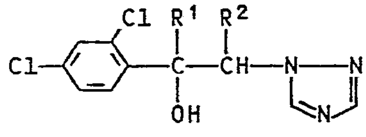

I,

in der
$R^1$ für einen $C_1$-$C_3$-Alkylrest und
$R^2$ für einen $C_1$-$C_8$-Alkylrest, für einen $C_3$-$C_8$-Cycloalkylrest, für einen $C_3$-$C_8$-Cycloalkyl-$C_1$-$C_3$-alkylrest, für gegebenenfalls substituiertes Benzyl oder Phenyl steht, wobei die asymmetrischen Zentren des Moleküls RR/SS bzw. threo-konfiguriert sind.

2. Verfahren zur Herstellung der Triazolylcarbinole der Formel I, dadurch gekennzeichnet, daß man ein Grignardreagenz der Formel II

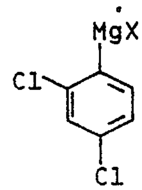

II

in der
X für Br oder J steht, umsetzt mit einem Triazolylketon der Formel III

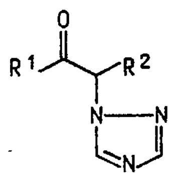

III,

in der $R^1$ und $R^2$ die oben angegebenen Bedeutungen haben.

3. Fungizides Mittel, gekennzeichnet durch einen Gehalt an einem Trägerstoff und einem weitgehend reinen Diastereomeren der Formel I

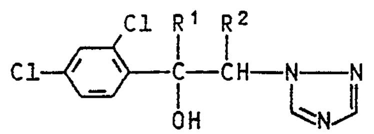

I,

in der
$R^1$ für einen $C_1$-$C_3$-Alkylrest und
$R^2$ für einen $C_1$-$C_8$-Alkylrest, für einen $C_3$-$C_8$-Cycloalkylrest, für einen $C_3$-$C_8$-Cycloalkyl-$C_1$-$C_3$-alkylrest, für gegebenenfalls substituiertes Benzyl oder Phenyl steht, wobei die asymmetrischen Zentren des Moleküls RR/SS bzw. threo-konfiguriert sind.

4. Pflanzenwachstumsregulierendes Mittel, gekennzeichnet durch einen Gehalt an einem Trägerstoff und einem weitgehend reinen Diastereomeren der Formel I

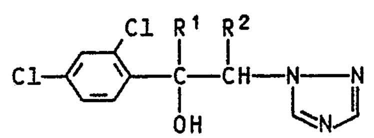

I,

in der
$R^1$ für einen $C_1$-$C_3$-Alkylrest und
$R^2$ für einen $C_1$-$C_8$-Alkylrest, für einen $C_3$-$C_8$-Cycloalkylrest, für einen $C_3$-$C_8$-Cycloalkyl-$C_1$-$C_3$-alkylrest, für gegebenenfalls substituiertes Benzyl oder Phenyl steht, wobei die asymmetrischen Zentren des Moleküls RR/SS bzw. threo-konfiguriert sind.

5. Weitgehend reine Threo-Diastereomere der Verbindungen 2-(2,4-Dichlorphenyl)-3-(1,2,4-triazol-1-yl)-butan-2-ol, 2-(2,4-Dichlorphenyl)-3-(1,2,4-triazol-1-yl)-pentan-2-ol.

6. Verfahren zur Bekämpfung von Pilzen, dadurch gekennzeichnet, daß man die Pilze oder die von Pilzbefall bedrohten Pflanzen, Materialien, Saatgüter oder den Boden mit einer fungizid wirksamen Menge einer Verbindung der Formel I

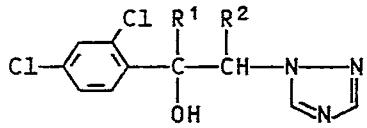

I,

in der

$R^1$ für einen $C_1$-$C_3$-Alkylrest und

$R^2$ für einen $C_1$-$C_8$-Alkylrest, für einen $C_3$-$C_8$-Cycloalkylrest, für einen $C_3$-$C_8$-Cycloalkyl-$C_1$-$C_3$-alkylrest, für gegebenenfalls substituiertes Benzyl oder Phenyl steht, wobei die asymmetrischen Zentren des Moleküls RR/SS bzw. threo-konfiguriert sind, behandelt.